# EUROPEAN PATENT SPECIFICATION

(11) **EP 3 810 001 B1**
(45) Date of publication and mention of the grant of the patent: **07.10.2026**
(21) Application number: 19735197.6
(22) Date of filing: 20.06.2019
(51) Int. Cl.: A61B 17/86

(54) **BONE SCREW IMPLANT FOR SACROILIAC JOINT FUSION**
KNOCHENSCHRAUBENIMPLANTAT FÜR ILIOSAKRALGELENKFUSION
IMPLANT À VIS À OS POUR FUSION D'ARTICULATION SACRO-ILIAQUE

(30) Priority: 20.06.2018 US 201862687339 P
(43) Date of publication of application: 28.04.2021
(73) Proprietor: Life Spine, Inc., Huntley, IL 60142 (US)
(72) Inventor: MEHL, David T., Lake in the Hills, IL 60156 (US); MESIWALA, Ali, H., Claremont, CA 91711 (US)
(74) Representative: V.O.
(86) International application number: PCT/US2019/038347
(87) International publication number: WO 2019/246468

(56) References cited:
- EP-A1- 3 123 970
- US-A1- 2013 018 427
- US-A1- 2015 012 051
- US-A1- 2016 287 301
- US-B1- 9 358 057

## Description

### FIELD OF THE INVENTION

The present invention relates to bone screws for the spine and related anatomy and, more particularly, to bone screws for sacroiliac joint fusion and related anatomy.

### BACKGROUND OF THE INVENTION

From the prior art multiple bone screw assemblies are known.

US 2016/287301 A1 e.g. discloses a bone screw, characterized by a two-piece compression screw assembly comprising a screw component and a sleeve component. The screw component has external male bone screw threading at a distal end, external male machine screw threading on a proximal end, and a smooth shank between the threaded distal and proximal ends. The screw component may be solid, cannulated, slotted, fenestrated, fluted, helically fluted, or any combination thereof. The sleeve component has external male bone screw threading with a pitch that can be equal to, smaller than, or greater than the thread pitch of the screw component, an internal bore extending from a proximal end of the sleeve component to the distal end of the sleeve component and sized for reception over the proximal end of the screw component. Internal female machine screw threading is configured to mate with the external male machine screw threading of the screw component.

US 2015/012051 A1 discloses a bone screw for sacroiliac joint stabilization. The fixation device includes an elongated body comprising a bone anchor at a distal end. An axially moveable proximal anchor is carried by the proximal end of the fixation device. In one embodiment, the device is inserted through the ilium of the pelvis and the bone anchor is rotated into position within the sacrum. The proximal anchor is distally advanced with respect to the bone anchor to provide compression across the sacroiliac joint.

EP 3 123 970 A1 discloses an implant, which enhances structural stability across a joint between a first and a second bone to be immobilized. The implant comprises a first and a second element, wherein the first element comprises a proximal, a distal and an intermediate section. The front end of the implant is formed as a pointed head with structures enabling the implant to penetrate bone material, including a channel-like structure running from the front end to a reservoir in the intermediate section. The reservoir is provided for receiving bone material through the channel-like structure and distributing it into a space between two bones upon placing the implant into the bones. A tool is provided for placing the implant into two bones, wherein the tool has means for connecting it to structures in the first element and the second element, so that the two elements can be moved independently.

US 9 358 057 B1 discloses a bone screw for transiliac fixation, which has an elongate hollow shaft. The hollow shaft has an externally threaded end portion extending to a tip end and a non-externally threaded shank portion having openings. The tip end has at least two bone cutting flutes at the bottom of the shaft. Each bone cutting flute has a cutting edge on a circumferential exterior of the threaded tip to cut bone and direct the cut bone internally into the hollow shaft toward the shank.

US 2013/018427 A1 discloses a bone screw assembly, that comprises a bone engagement portion including a shaft and a head member. The assembly also includes a flexible washer member that can be operably attached and secured around the head member of the bone engagement portion. The washer member is polyaxial relative to the bone engagement portion, such that it can assume a variety of angles. The washer member can help prevent back out of the bone engagement portion when the bone engagement portion is implanted into a bone member. The design of the fixation screw assembly can be used in different fusion procedures, including fusion of the sacroiliac joint.

Bone screws are used routinely for repairing, stabilizing, fixing, fusing and other orthopedic purposes of bones of the body such as the spine and pelvis. However, no matter where bone screws are used, they face challenges of "backing out" of the anatomy in which they are installed. Bone screw malfunction such as backing out can compromise the integrity of the orthopedic purpose for which the bone screws were installed and/or pose serious risks and/or complications for the implant recipient.

In view of the above, it is therefore desirous to have a bone screw that can minimize or eliminate backing out.

### SUMMARY OF THE INVENTION

According to the invention, a bone screw implant is provided for sacroiliac joint fusion comprising: a bone screw defining a longitudinal axis and having external threading, a proximal end having sleeve engagement geometry, a distal end having a tip with a self-tapping geometry, the external threading extending from the distal end to proximate the proximal end, and a self-harvesting bone cutting geometry about the longitudinal axis that gathers and retains bone shavings produced by the bone cutting geometry; and a cylindrical sleeve having a generally cylindrical body, an external surface, a first end, a second end, a cylindrical bore extending from the first end to the second end and defining an internal surface, anti-rotation geometry on the external surface, and bone screw engagement geometry on the internal surface; wherein the self-harvesting bone cutting geometry comprises helical cutting flutes extending through the external threading, the helical cutting flutes configured to produce the bone graft during implantation of the bone screw; wherein the anti-rotation geometry of the sleeve comprises a plurality of tapered fins at the outside of the body, and wherein each fin projects radially outward from and extends axially along the outer surface of the body.

A bone screw implant for sacroiliac joint fusion includes a bone screw and a self-contained rectilinear sleeve, the sleeve configured to resist reverse rotation of the bone screw once installed to help prevent screw back-out, the bone screw having self-drilling tip geometry that allows the bone screw to be installed without need for a pilot hole, and self-harvesting geometry that gathers and retains bone shavings generated by bone screw installation in the bone screw for use as graft for bone fusion.

The sleeve has non-radial (axial) geometry on its external surface that aids in resisting rotational forces exerted on the implant when fully seated into the bone (in situ). The anti-rotation geometry press fits linearly into the bony anatomy surrounding the bone screw to fix its rotational position. The anti-rotation geometry of the sleeve comprises tapered fins. In another form, the anti-rotational geometry comprises tapered flutes. In yet another form, the anti-rotational geometry comprises non-tapered flutes. Other geometries are contemplated.

The sleeve also has geometry that provides permanent assembly onto the bone screw and which binds/connects itself to/at a specific axial position on the bone screw to help prevent bone screw back-out. In one form, the sleeve has an inner radial groove within its inner diameter that engages a circumferential projection (e.g. ring) on the bone screw that allows the sleeve to be captured by the bone screw, while also allowing the sleeve to freely rotate with respect to the bone screw.

The freely rotating sleeve allows the sleeve to be press-fit linearly into the bone while the bone screw threads into the bone until both components are fully seated into the bone. In order for the bone screw to back out of the bone, the threads would have to rotationally loosen while also overcoming shear forces due to the presence of the press-fit condition/connection of the sleeve to the end of the bone screw.

The bone screw may include external threads on its proximal end for interfacing with a screwdriver. The proximal threads provide a secure attachment interface with the screwdriver so that the bone screw does not inadvertently disengage from the screwdriver while installing the bone screw into the bone.

The sleeve may additionally include threads on its inner surface proximate to the proximal end of the sleeve that engage the external threads on the proximal end of the bone screw. The combination of these thread forms on both components are coupled with a set screw for a secondary mechanism locking the two components together once the bone screw has been fully seated within the bone. The set screw would further prevent the bone screw from backing out and "pushing" the sleeve linearly out of the bone.

The self-drilling bone screw tip geometry is characterized by a plurality of cutting tips or blades situated about a central opening. This geometry allows the surgeon to eliminate operative steps, thus shortening surgical time.

The self-harvesting geometry is characterized by helical cutting flutes and windows situated at various intervals within the helical flute geometry that harvest bone/bone shavings (autograft) during insertion of the bone screw into the boney anatomy. Particularly, as the bone screw is rotated into the boney anatomy, the helical cutting flutes gather and deposit bone shavings into the windows. In one form, the windows comprise round and/or oval holes. In another form, the windows comprise slots. In all cases, the windows can be perpendicular with respect to the long axis of the bone screw, angular with respect to the long axis of the bone screw, formed in a helical pattern with respect to the helical trajectory of the helical cutting flutes, and/or a combination thereof. This feature solves the current challenges of pre-packing the bone screw/implant with graft biologic prior to implantation or inserting graft biologic into the bone screw/implant through an axial cannula of the bone screw after final implantation.

### BRIEF DESCRIPTION OF THE DRAWING FIGURES

The features of the invention will be better understood by reference to the accompanying drawings, wherein:
Fig. 1 is an isometric view of a bone screw implant fashioned in accordance with the principles of the present invention;
Fig. 2 is an exploded isometric view of the bone screw implant of Fig. 1;
Fig. 3 is an exploded side view of the bone screw implant of Fig. 1;
Fig. 4 is an exploded side sectional view of the bone screw implant of Fig. 1;
Fig. 5 is another isometric view of the bone screw implant of Fig. 1;
Fig. 6 is an isometric sectional view of the bone screw implant of Fig. 1;
Fig. 7 is an enlarged sectional view of the proximal end of the bone screw and sleeve of the bone screw implant of Fig. 1;
Fig. 8 is a side view of the sleeve of the bone screw implant of Fig. 1;
Fig. 9 is an end view of the sleeve of the bone screw implant of Fig. 1;
Fig. 10 is an isometric view of the sleeve of the bone screw implant of Fig. 1;
Fig. 11 is an enlarged view of the proximal end of the bone screw of the bone screw implant of Fig. 1;
Fig. 12 is an isometric view of an alternate sleeve for the bone screw implant of Fig. 1;
Fig. 13 is an end view of the alternate sleeve of Fig. 12;
Fig. 14 is a side view of the alternate sleeve of Fig. 12;
Fig. 15 is an isometric view of the bone screw of the bone screw implant of Fig. 1;
Fig. 16 is an enlarged view of the distal end (tip) of the bone screw of the bone screw implant of Fig. 1;
Fig. 17 is an end view of the distal end of the bone screw of the bone screw implant of Fig. 1;
Fig. 18 is an enlarged isometric view of the bone screw of the bone screw implant of Fig. 1;
Fig. 19 is an enlarged end view of the distal end (tip) of the bone screw of the bone screw implant of Fig. 1;
Fig. 20 is an enlarged side view of the bone screw of the bone screw implant of Fig. 1;
Fig. 21 is an isometric view of another bone screw implant fashioned in accordance with the present principles;
Fig. 22 is an enlarged view of the proximal end of the bone screw implant of Fig. 21;
Fig. 23 is a sectional view of the bone screw implant of Fig. 21 taken along line 23-23 thereof;
Fig. 24 is a sectional view of the proximal end of the bone screw implant of Fig. 22 taken along line 24-24 thereof;
Fig. 25 is another sectional view of the bone screw implant of Fig. 21;
Fig. 26 is another sectional view of the proximal end of the bone screw implant of Fig. 21;
Fig. 27 is a sectional view of the proximal end of the bone screw implant of Fig. 21 without the set screw;
Fig. 28 is a sectional view of the proximal end of the bone screw implant of Fig. 21 with the set screw shown exploded therefrom;
Fig. 29 is another sectional view of the proximal end of the bone screw implant of Fig. 21 with the set screw shown exploded therefrom; and
Fig. 30 is an enlarged view of the proximal end of the bone screw implant of Fig. 21 with the set screw shown exploded therefrom.

### DETAILED DESCRIPTION OF THE INVENTION

For the purposes of promoting an understanding of the principles of the present disclosure, reference will now be made to the embodiments illustrated in the drawings, and specific language will be used to describe the same. It will nevertheless be understood that no limitation of the scope of this database is thereby intended.

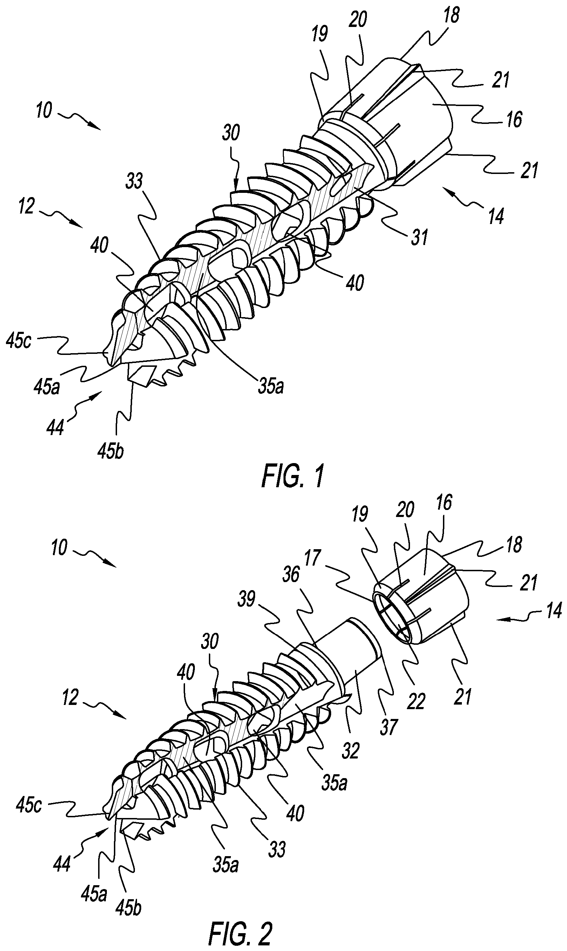

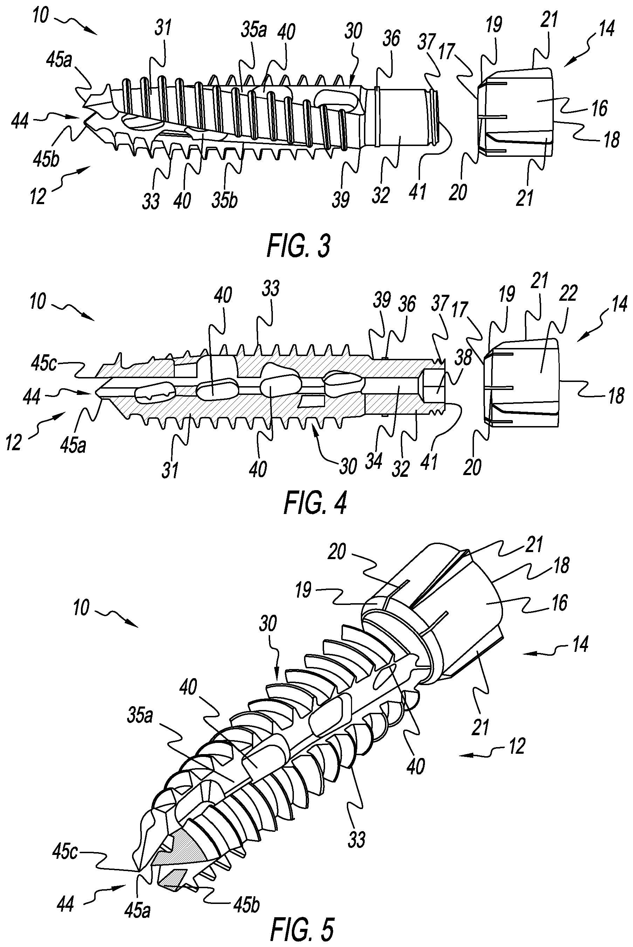

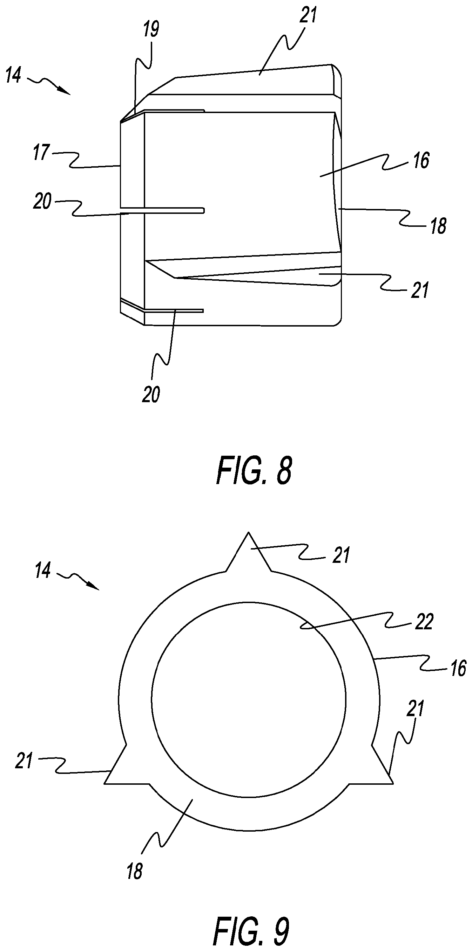

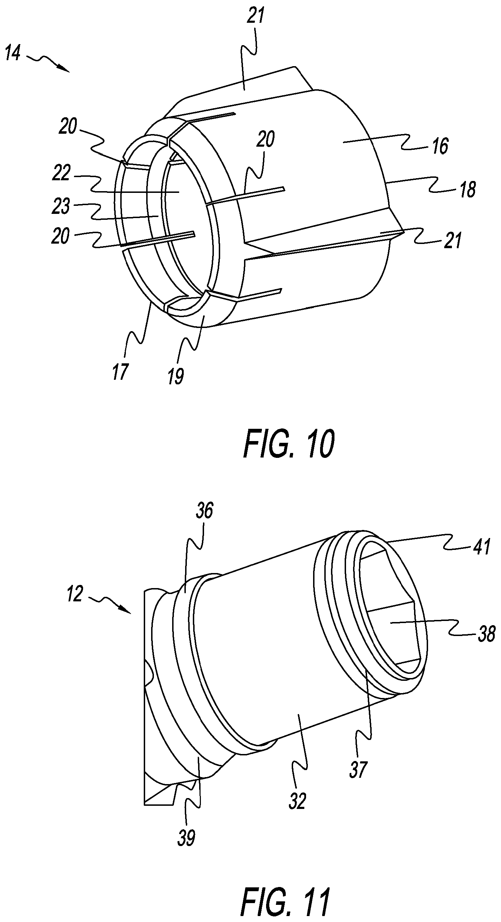

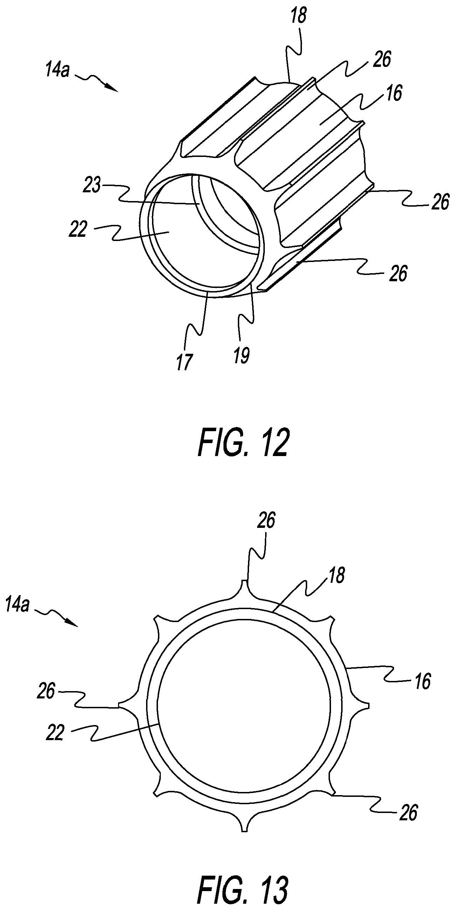

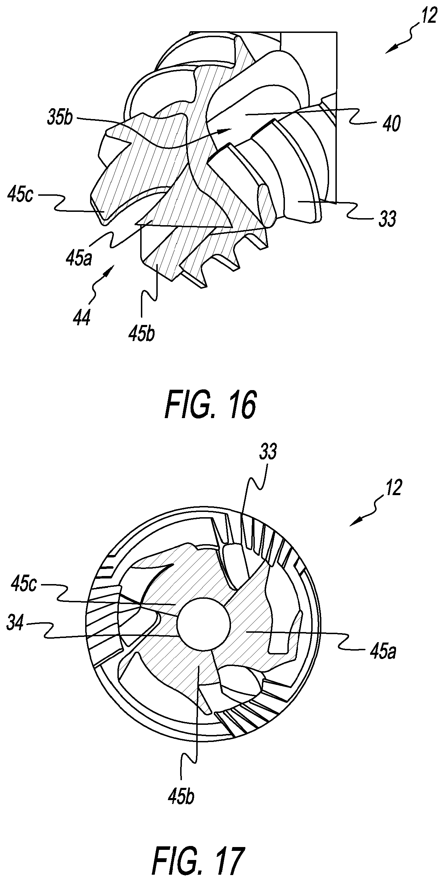

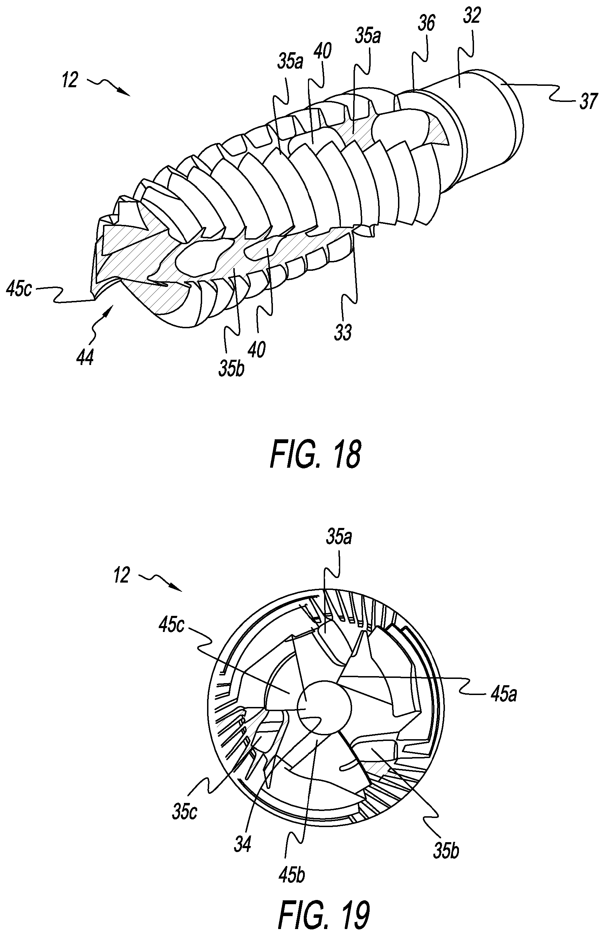

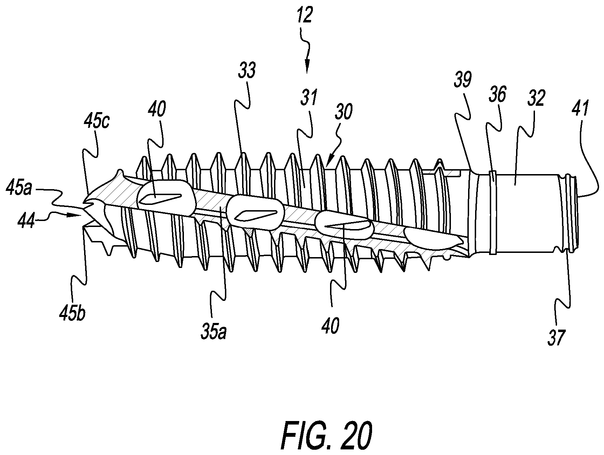

Figs. 1-7 depict various exploded and non-exploded views of a bone screw implant 10 fashioned in accordance with the present principles, characterized by a bone screw 12 and a sleeve 14. Figs. 11 and 15-20 depict various views of the bone screw 12. Figs. 8-10 depict various views of the sleeve 14, while Figs. 12-14 depict various views of an alternate sleeve 14a. The bone screw implant 10 is best used for sacroiliac fusion, but may be used for various spine applications as well as other orthopedic applications.

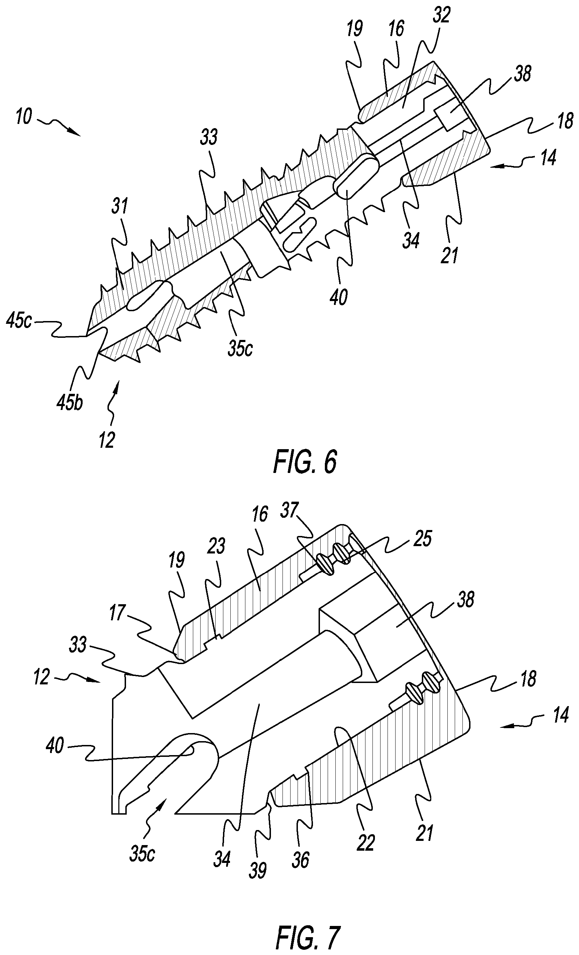

The bone screw 12 is characterized by a body 30 having a self-drilling or self-tapping tip 44 at its distal end and may include external threading 37 adjacent the proximal end 41 of the body 30, and particularly situated about an upper portion of the unthreaded tubular end section 32. The body 30 defines a longitudinal or long axis and length between the tip 44 and the end 41 and includes external threads/threading 33 on an outer surface thereof extending generally from the tip/distal end 44 to the end section 32. An internal cannula 34 preferably, but not necessarily, extends the entire longitudinal length of the body 30, and intersects with a socket 38 in the upper cylindrical section 32 of the body 30, providing communication between the tip 44 and the socket 38. The socket 38 is configured to receive a like-configured tool (not shown) for use in implanting the bone screw implant 10. The external surface of the upper section 32 of the body 30 has a ridge, projection, annulus, ring or the like (collectively, ring) 36 that extends about the circumference of the upper section 32 proximate a neck 39 between the upper section 32 and the external threading 33 of the body. The ring 36 may be continuous (congruent) as shown, or may be discontinuous (non-congruent) such as is formed by a plurality of ring sections (not shown). The ring 36 engages an inner radial groove 23 in an inner diameter 22 of the sleeve 14 (see, e.g. Fig. 7) the co-action allowing the sleeve 14 to be captured by the bone screw 12.

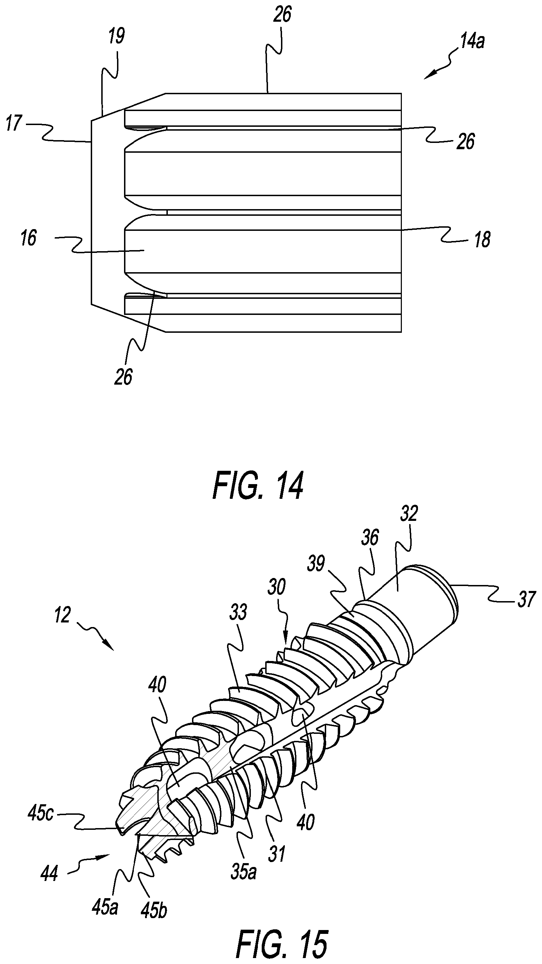

The tip 44 of the bone screw 12 (see, e.g. Figs. 15-20) is self-drilling, and is formed of several (e.g. a plurality of) cutting blades, heads or the like 45a, 45b, 45c, it being understood that the tip 44 may comprise more or less cutting points 45. The cutting blades 45a-c are situated about an opening of the cannula 34 at the tip 44, and are preferably, but not necessarily, equally radially spaced about the cannula opening. Each blade 45a-c is configured to drill, cut or otherwise dig into bone.

The bone screw 12 has self-harvesting geometry that gathers and retains bone shavings generated by bone screw installation for use as graft for bone fusion. To this end, the bone screw 12 has several (e.g. a plurality of) preferably, but not necessarily, helical cutting channels, grooves, flutes or the like (collectively, flutes) 35a, 35b, 35c extending through the threading 33 of the bone screw body 30 from the tip 44 to the upper section 32. One helical cutting flute 35 is associated with one cutting blade 45, such that the cutting blade 45a is associated with the helical cutting flute 35a, the cutting blade 45b is associated with the helical cutting flute 35b, and the cutting blade 45c is associated with the helical cutting flute 35c. Each cutting flute 35 has a plurality of windows, openings, holes or the like (collectively, windows) 40 situated at various intervals within the helical flute geometry that together harvest bone/bone shavings (autograft) during insertion of the bone screw into the boney anatomy. Particularly, as the bone screw 12 is rotated into the boney anatomy, the helical cutting flutes 35 gather and deposit bone shavings into the windows 40. In one form, the windows 40 are round and/or oval holes. In another form, the windows 40 are slots. In all cases, the windows 40 can be perpendicular with respect to the long axis of the bone screw, angular with respect to the long axis of the bone screw, formed in a helical pattern with respect to the helical trajectory of the helical cutting flutes, and/or a combination thereof.

The sleeve 14 is characterized by a generally cylindrical body 16 defining a front 17, an end 18, and an interior, bore, or the like 22. The front 17 of the body 16 has a taper, angle, or slant (collectively, taper) 19 that circumferentially surrounds the front 17, the taper 19 providing a transition from the body 30 of the bone screw 12 to the sleeve 14 for ease in installation/implantation. The sleeve 14 has an anti-rotation feature that performs the function of resisting rotation of the implant (screw assembly) 10 when fully seated into the bone, the anti-rotation feature comprising three (3) fins or the like 21 on the outside of the body 16, each fin 21 projecting radially outward from and extending axially along the outer surface of the body 16. Each fin 21 is preferably, but not necessarily, tapered from the front thereof (proximate the front 17 of the body 16) to the rear thereof (at or proximate the rear 18 of the body 16). The external fins 21 are also preferably, but not necessarily, equally spaced about the outer circumference of the body 16. It should be appreciated that in all instances, the number of external fins 21 may be more or less than three (3). The body 16 further has a plurality of slits, slots, channels or the like (collectively, slits) 20 that are situated about the radial circumference/diameter of the body at the front 17 thereof. Each slit 20 extends axially along the body 16 from the front 17 to a distance into the body. The slits 20 allow resilient expansion (then contraction) of the front portion of the sleeve 14 so that the ring 36 of the bone screw 12 can be received in the groove 23 of the sleeve 14 in a snap-fit fashion.

The inside of the sleeve 14 has a radial groove, slot, channel, cutout or the like (collectively, groove) 23 that preferably, but not necessarily, extends the full circumference or diameter about or around the inner surface of the bore 22 proximate the front 17. The radial groove 23 is sized to receive the ring 36 of the bone screw 12. Particularly, the radial groove 23 of the sleeve 14 receives the ring 36 of the bone screw 12 such that the bone screw 12 captures the sleeve 14 while also allowing the sleeve 14 to freely rotate with respect to the bone screw 12. The freely rotating sleeve allows the sleeve to be press-fit linearly into a receiving bone (not shown) while the bone screw threads into the receiving bone until both components are fully seated in the receiving bone.

Figs. 12-14 depict an alternative sleeve 14a for the bone screw 12/implant 10. The sleeve 14a is a variation of the sleeve 14. Particularly, the sleeve 14a has a variation of its anti-rotation feature. However, other features, components and the like are the same as the sleeve 14 and therefore such same and/or similar features, components and the like have the same call-out number as the sleeve 14. The sleeve 14a has a plurality of flutes or the like 26 on the outside of the body 16, each flute 26 projecting radially outward from and extending axially along the outer surface of the body 16. Each flute 26 may be tapered from the front thereof (proximate the front 17 of the body 16) to the rear thereof (at or proximate the rear 18 of the body 16) or not. The external flutes 26 are also preferably, but not necessarily, equally spaced about the outer circumference of the body 16. It should be appreciated that in all instances, the number of external flutes 26 may be more or less than that shown.

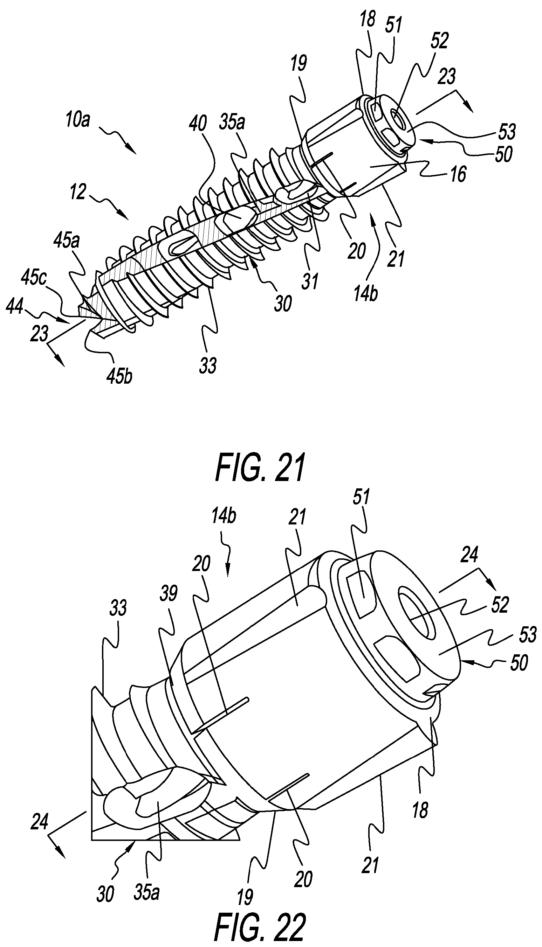

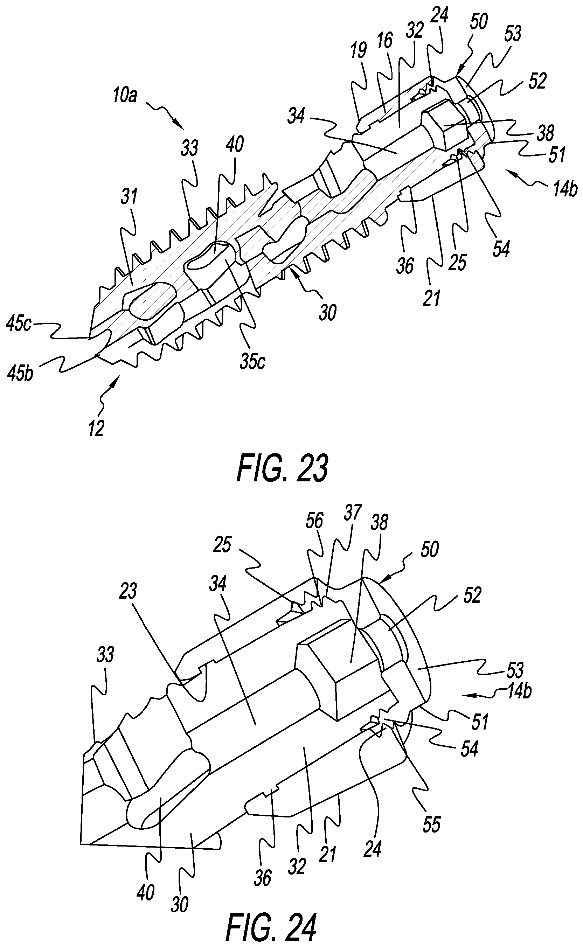

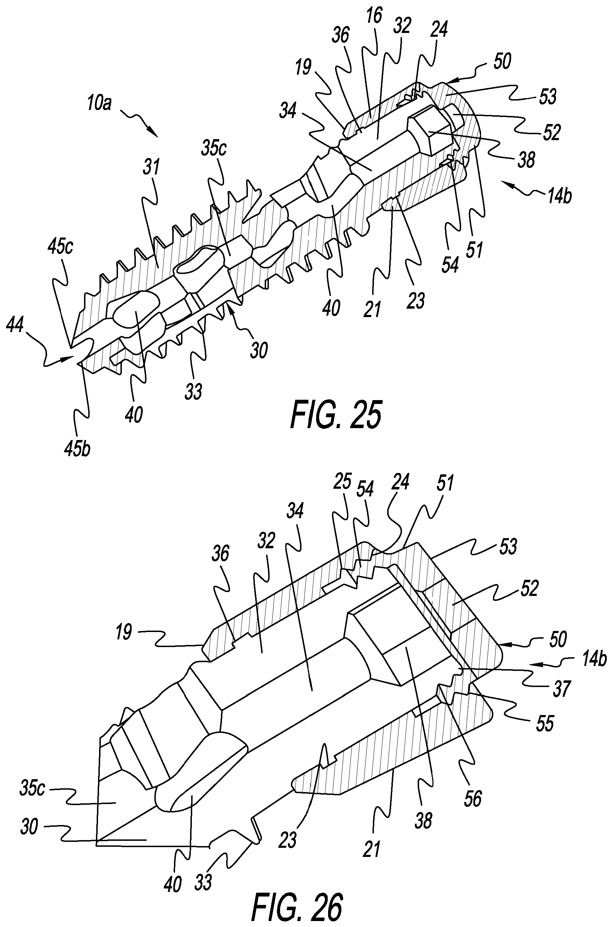

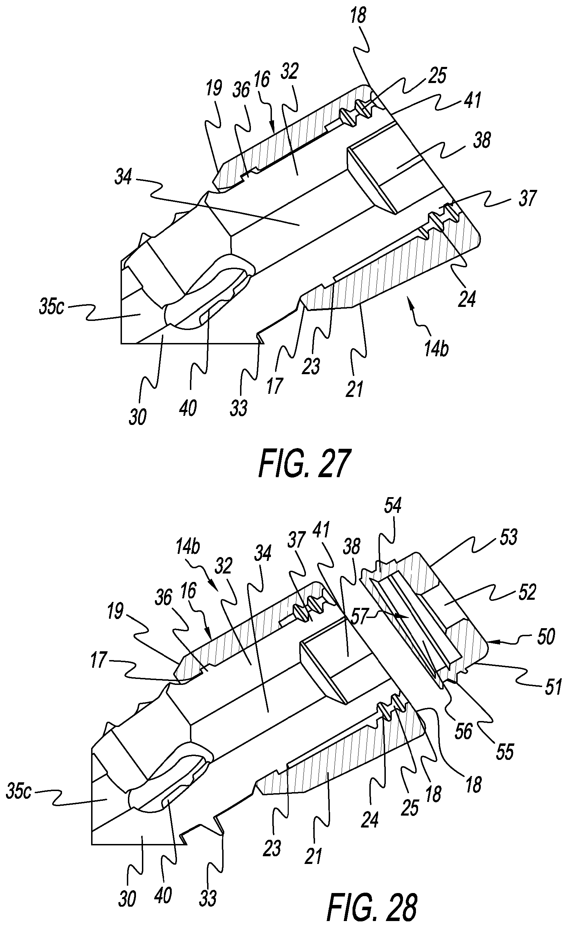

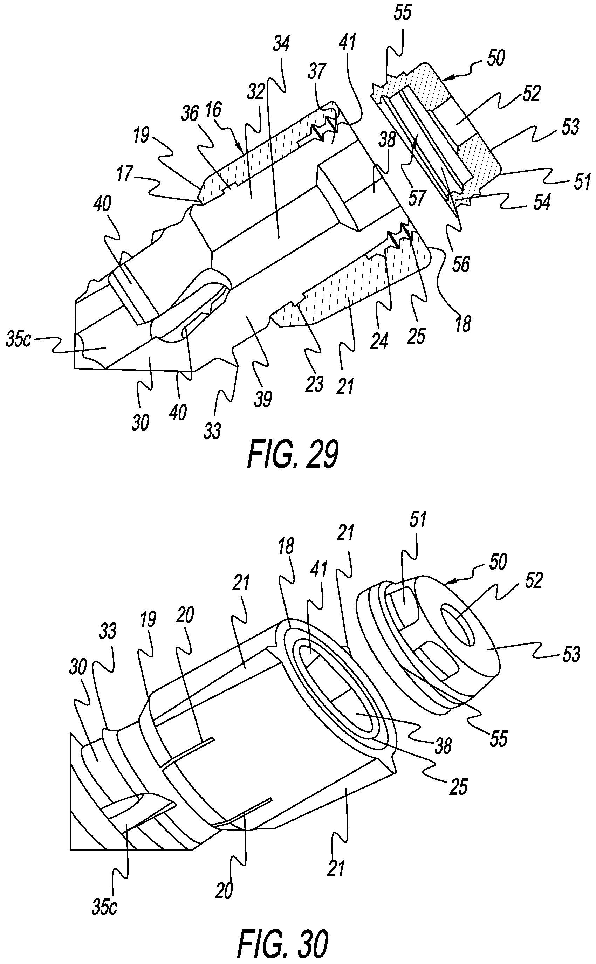

Figs. 21-30 depict various views of another bone screw implant 10a fashioned in accordance with the present principles, characterized by the bone screw 12, a sleeve 14b and a set screw 50 (the sleeve and set screw forming a sleeve assembly). The bone screw implant 10a is the same as the bone screw implant 10 except for the incorporation of a secondary locking mechanism between the bone screw 12 and the sleeve/sleeve assembly 14b. As such, the features, characteristics, properties, and attributes of the bone screw 12 will not be re-discussed except where necessary and/or appropriate in describing the bone screw assembly 10a such as, but not limited to, describing the bone screw 12 relative to the sleeve/sleeve assembly 14b.

The sleeve 14b is the same as the sleeve 14 with the exception of the addition of threading 24 on its upper inside wall/bore 22 proximate the end 18. The bore 22 may be sized slightly larger than the bore 22 of the bone screw implant 10 to form an inner thread cavity 25 or its inner cavity may be sized the same. The sleeve assembly includes a set screw 50 that provides secondary locking of the sleeve 14b to the bone screw 12. The set screw 50 has a hexagonal side 51 (or other configuration for engagement with a set screw driver [not shown]). A bore 52 is disposed in the top 53 of the set screw 50 that allows engagement with an installation/de-installation tool (not shown). The set screw 50 also has a lower cylindrical wall 54 defining a cylindrical cavity 57. Threading 55 is provided on the outside of the annular wall 54. Threading 56 is also provided on the inside of the annular wall 54.

Once the sleeve 14b has been installed on the bone screw 12, the set screw 50 is installed. The set screw 50 is threaded into the annular cavity 25 formed between the outside of the upper section 32 of the bone screw 12 and the inside of the upper area of the sleeve 14b. Particularly, the annular wall 54 of the set screw 50 is received in the annular cavity 25. The inner threading 56 of the annular wall 54 of the set screw 50 engages the outer threading 37 of the bone screw 12, while the outer threading 55 of the annular wall 54 of the set screw 50 engages the inner threading 24 of the sleeve 14b. This couples the sleeve to the bone screw.

It should be readily grasped that the present bone screw implant is advantageous over the current state of the art.

## Claims

1. A bone screw implant (10, 10a) for sacroiliac joint fusion comprising:
a bone screw (12) defining a longitudinal axis and having external threading (33), a proximal end (41) having sleeve engagement geometry, a distal end (44) having a tip with a self-tapping geometry, the external threading (33) extending from the distal end (44) to proximate the proximal end (41), and
a self-harvesting bone cutting geometry about the longitudinal axis that gathers and retains bone shavings produced by the bone cutting geometry; and
a cylindrical sleeve (14, 14a, 14b) having a generally cylindrical body (16), an external surface, a first end (17), a second end (18), a cylindrical bore (22) extending from the first end (17) to the second end (18) and defining an internal surface, anti-rotation geometry on the external surface, and bone screw engagement geometry on the internal surface;
**characterized in that**
the self-harvesting bone cutting geometry comprises helical cutting flutes extending through the external threading (33), the helical cutting flutes configured to produce the bone graft during implantation of the bone screw;
wherein the anti-rotation geometry of the sleeve (14, 14a, 14b) comprises a plurality of tapered fins (21) at the outside of the body (16), and wherein each fin (21) projects radially outward from and extends axially along the outer surface of the body (16).

2. The bone screw implant of claim 1, wherein the self-tapping geometry of the tip (44) comprises a plurality of cutting blades (45a-c) arranged about a central hole.

3. The bone screw implant of claim 1, further comprising a plurality of windows (40) in the helical cutting flutes (35a, 35b, 35c), each one of the plurality of windows (40) configured to receive and hold the bone shavings produced by the helical cutting flutes (35a, 35b, 35c) during implantation of the bone screw (12).

4. The bone screw implant of claim 3, wherein the plurality of windows (40) are situated at various intervals about each helical cutting flute (35a, 35b, 35c).

5. The bone screw implant of claim 4, wherein the windows (40) comprise round and/or oval holes or comprise slots.

6. The bone screw implant of claim 4, wherein the plurality of windows (40) are perpendicular with respect to the longitudinal axis, or are angular with respect to the longitudinal axis.

7. The bone screw implant of claim 1, wherein:
the sleeve engagement geometry of the bone screw (12) comprises a circumferential projection; and
the bone screw engagement geometry of the sleeve (14) comprises a radial groove (23) sized to receive the circumferential projection in a press-fit manner.

8. The bone screw implant of claim 7, wherein:
the radial projection comprises a circumferential ridge (36); and
the radial groove (23) comprises a circumferential channel.

9. The bone screw implant of claim 8, wherein:
the sleeve engagement geometry of the proximal end (41) of the bone screw (12) further comprises first radial threading (37) at an end of the proximal end (41); and
the bone screw engagement geometry of the sleeve (14) further comprises second radial threading (24) at a distal end of the sleeve (14).

10. The bone screw implant of claim 9, further comprising a set screw (50) having:
external threading (55) that provides engagement with the second radial threading (24) ; and
internal threading (56) that provides engagement with the first radial threading (37).

11. The bone screw implant of claim 1, wherein the plurality of fins (21) are equally spaced about the external surface of the sleeve (14).

12. The bone screw implant of claim 1, wherein each fin (21) is tapered from the first end (17) to the second end (18).

13. The bone screw implant of claim 1, wherein the plurality of fins (21) comprises three fins.

14. The bone screw implant of claim 1, wherein the sleeve (14) further comprises a plurality of slits (20) situated about the radial circumference of the first end (17) of the sleeve (14).

15. The bone screw implant of claim 14, wherein each slit (20) extends axially along the sleeve from the first end (17) to a distance toward the second end (18).

16. The bone screw implant of claim 15, wherein the plurality of slits(20) are configured to allow resilient expansion and contraction of the first end (17) of the sleeve (14).

## Patentansprüche

1. Knochenschraubenimplantat (10, 10a) zur Fusion des Sakroiliakalgelenks, umfassend:
eine Knochenschraube (12), die eine Längsachse definiert und ein Außengewinde (33), ein proximales Ende (41) mit Hülseneingriffsgeometrie und ein distales Ende (44) mit einer Spitze mit einer selbstschneidenden Geometrie aufweist, wobei sich das Außengewinde (33) so vom distalen Ende (44) erstreckt, dass es sich dem proximalen Ende (41) annähert, und
eine selbstentnehmende Knochenschneidgeometrie um die Längsachse, die Knochenspäne, die durch die Knochenschneidgeometrie erzeugt werden, erfasst und zurückhält; und
eine zylindrische Hülse (14, 14a, 14b) mit einem allgemein zylindrischen Körper (16), eine Außenfläche, ein erstes Ende (17), ein zweites Ende (18), eine zylindrische Bohrung (22), die sich vom ersten Ende (17) zum zweiten Ende (18) erstreckt und eine Innenfläche definiert, Antirotationsgeometrie an der Außenfläche, und Knochenschraubeneingriffsgeometrie an der Innenfläche;
**dadurch gekennzeichnet, dass**
die selbstentnehmende Knochenschneidgeometrie spiralförmige Schneidnuten umfasst, die sich durch das Außengewinde (33) erstrecken, wobei die spiralförmigen Schneidnuten dazu ausgestaltet sind, während der Implantation der Knochenschraube das Knochentransplantat zu erzeugen;
wobei die Antirotationsgeometrie der Hülse (14, 14a, 14b) eine Mehrzahl an abgeschrägten Lamellen (21) an der Außenseite des Körpers (16) umfasst und wobei jede Lamelle (21) radial nach außen von der Außenfläche des Körpers (16) vorsteht und sich axial entlang dieser erstreckt.

2. Knochenschraubenimplantat nach Anspruch 1, wobei die selbstschneidende Geometrie der Spitze (44) eine Mehrzahl an um ein zentrales Loch angeordneten Schneidklingen (45a-c) umfasst.

3. Knochenschraubenimplantat nach Anspruch 1, ferner umfassend eine Mehrzahl an Fenstern (40) in den spiralförmigen Schneidnuten (35a, 35b, 35c), wobei jedes von der Mehrzahl an Fenstern (40) dazu ausgestaltet ist, die von den spiralförmigen Schneidnuten (35a, 35b, 35c) während der Implantation der Knochenschraube (12) erzeugten Knochenspäne aufzunehmen und zu halten.

4. Knochenschraubenimplantat nach Anspruch 3, wobei die Mehrzahl an Fenstern (40) in verschiedenen Abständen um jede spiralförmige Schneidnut (35a, 35b, 35c) angeordnet ist.

5. Knochenschraubenimplantat nach Anspruch 4, wobei die Fenster (40) runde und/oder ovale Löcher umfassen oder Schlitze umfassen.

6. Knochenschraubenimplantat nach Anspruch 4, wobei die Mehrzahl an Fenstern (40) senkrecht zur Längsachse sind oder winklig zur Längsachse sind.

7. Knochenschraubenimplantat nach Anspruch 1, wobei:
die Hülseneingriffsgeometrie der Knochenschraube (12) einen umlaufenden Vorsprung umfasst; und
die Knochenschraubeneingriffsgeometrie der Hülse (14) eine radiale Nut (23) umfasst, die so dimensioniert ist, dass sie den umlaufenden Vorsprung in der Art eines Presssitzes aufnimmt.

8. Knochenschraubenimplantat nach Anspruch 7, wobei:
der radiale Vorsprung eine umlaufende Erhöhung (36) umfasst; und
die radiale Nut (23) einen umlaufenden Kanal umfasst.

9. Knochenschraubenimplantat nach Anspruch 8, wobei:
die Hülseneingriffsgeometrie des proximalen Endes (41) der Knochenschraube (12) ferner ein erstes radiales Gewinde (37) an einem Ende des proximalen Endes (41) umfasst; und
die Knochenschraubeneingriffsgeometrie der Hülse (14) ferner ein zweites radiales Gewinde (24) an einem distalen Ende der Hülse (14) umfasst.

10. Knochenschraubenimplantat nach Anspruch 9, ferner umfassend eine Stellschraube (50), aufweisend:
ein Außengewinde (55), das ein Eingreifen in das zweite radiale Gewinde (24) ermöglicht; und
ein Innengewinde (56), das ein Eingreifen in das erste radiale Gewinde (37) ermöglicht.

11. Knochenschraubenimplantat nach Anspruch 1, wobei die Mehrzahl an Lamellen (21) gleichmäßig um die Außenfläche der Hülse (14) beabstandet sind.

12. Knochenschraubenimplantat nach Anspruch 1, wobei jede Lamelle (21) vom ersten Ende (17) zum zweiten Ende (18) verjüngt ist.

13. Knochenschraubenimplantat nach Anspruch 1, wobei die Mehrzahl an Lamellen (21) drei Lamellen umfasst.

14. Knochenschraubenimplantat nach Anspruch 1, wobei die Hülse (14) ferner eine Mehrzahl an Schlitzen (20) umfasst, die sich um den radialen Umfang des ersten Endes (17) der Hülse (14) befinden.

15. Knochenschraubenimplantat nach Anspruch 14, wobei sich jeder Schlitz (20) axial entlang der Hülse vom ersten Ende (17) bis zu einem Abstand in Richtung des zweiten Endes (18) erstreckt.

16. Knochenschraubenimplantat nach Anspruch 15, wobei die Mehrzahl an Spalten (20) dazu ausgestaltet ist, eine elastische Expansion und Kontraktion des ersten Endes (17) der Hülse (14) zu erlauben.

## Revendications

1. Implant à vis à os (10, 10a) pour la fusion de l'articulation sacro-iliaque comprenant :
une vis à os (12) définissant un axe longitudinal et ayant un filetage externe (33), une extrémité proximale (41) ayant une géométrie de mise en prise de manchon, une extrémité distale (44) ayant une pointe avec une géométrie autotaraudeuse, le filetage externe (33) s'étendant de l'extrémité distale (44) à proximité de l'extrémité proximale (41), et
une géométrie de coupe d'os auto-récoltante autour de l'axe longitudinal qui recueille et retient les copeaux osseux produits par la géométrie de coupe d'os ; et
un manchon cylindrique (14, 14a, 14b) ayant un corps généralement cylindrique (16), une surface externe, une première extrémité (17), une deuxième extrémité (18), un alésage cylindrique (22) s'étendant de la première extrémité (17) à la deuxième extrémité (18) et définissant une surface interne, une géométrie anti-rotation sur la surface externe et une géométrie de mise en prise de vis à os sur la surface interne ;
**caractérisé en ce que** :
la géométrie de coupe d'os auto-récoltante comprend des cannelures de coupe hélicoïdales s'étendant à travers le filetage externe (33), les cannelures de coupe hélicoïdales étant configurées pour produire la greffe osseuse pendant l'implantation de la vis à os ;
dans lequel la géométrie anti-rotation du manchon (14, 14a, 14b) comprend une pluralité d'ailettes progressivement rétrécies (21) à l'extérieur du corps (16), et dans lequel chaque ailette (21) fait saillie radialement vers l'extérieur à partir de et s'étend axialement le long de la surface externe du corps (16).

2. Implant à vis à os selon la revendication 1, dans lequel la géométrie autotaraudeuse de la pointe (44) comprend une pluralité de lames de coupe (45a-c) agencées autour d'un trou central.

3. Implant à vis à os selon la revendication 1, comprenant en outre une pluralité de fenêtres (40) dans les cannelures de coupe hélicoïdales (35a, 35b, 35c), chacune de la pluralité de fenêtres (40) étant configurée pour recevoir et maintenir les copeaux d'os produits par les cannelures de coupe hélicoïdales (35a, 35b, 35c) pendant l'implantation de la vis à os (12).

4. Implant à vis à os selon la revendication 3, dans lequel la pluralité de fenêtres (40) sont situées à différents intervalles autour de chaque cannelure de coupe hélicoïdale (35a, 35b, 35c).

5. Implant à vis à os selon la revendication 4, dans lequel les fenêtres (40) comprennent des trous ronds et/ou ovales ou comprennent des fentes.

6. Implant à vis à os selon la revendication 4, dans lequel la pluralité de fenêtres (40) sont perpendiculaires par rapport à l'axe longitudinal, ou sont angulaires par rapport à l'axe longitudinal.

7. Implant à vis à os selon la revendication 1, dans lequel :
la géométrie de mise en prise de manchon de la vis à os (12) comprend une saillie circonférentielle ; et
la géométrie de mise en prise de vis à os du manchon (14) comprend une rainure radiale (23) dimensionnée pour recevoir la saillie circonférentielle d'une manière ajustée par serrage.

8. Implant à vis à os selon la revendication 7, dans lequel :
la saillie radiale comprend une crête circonférentielle (36) ; et
la rainure radiale (23) comprend un canal circonférentiel.

9. Implant à vis à os selon la revendication 8, dans lequel :
la géométrie de mise en prise de manchon de l'extrémité proximale (41) de la vis à os (12) comprend en outre un premier filetage radial (37) à une extrémité de l'extrémité proximale (41) ; et
la géométrie de mise en prise de vis à os du manchon (14) comprend en outre un deuxième filetage radial (24) à une extrémité distale du manchon (14).

10. Implant à vis à os selon la revendication 9, comprenant en outre une vis de réglage (50) ayant :
un filetage externe (55) qui fournit la mise en prise avec le deuxième filetage radial (24) ; et
un filetage interne (56) qui fournit la mise en prise avec le premier filetage radial (37).

11. Implant à vis à os selon la revendication 1, dans lequel la pluralité d'ailettes (21) sont espacées à égale distance autour de la surface externe du manchon (14).

12. Implant à vis à os selon la revendication 1, dans lequel chaque ailette (21) est progressivement rétrécie de la première extrémité (17) à la deuxième extrémité (18).

13. Implant à vis à os selon la revendication 1, dans lequel la pluralité d'ailettes (21) comprend trois ailettes.

14. Implant à vis à os selon la revendication 1, dans lequel le manchon (14) comprend en outre une pluralité de fentes (20) situées autour de la circonférence radiale de la première extrémité (17) du manchon (14).

15. Implant à vis à os selon la revendication 14, dans lequel chaque fente (20) s'étend, de manière axiale, le long du manchon à partir de la première extrémité (17) sur une distance vers la deuxième extrémité (18).

16. Implant à vis à os selon la revendication 15, dans lequel la pluralité de fentes (20) sont configurées pour permettre l'expansion et la contraction résiliente de la première extrémité (17) du manchon (14).
